# EUROPÄISCHE PATENTSCHRIFT

(11) **EP 4 000 526 B1**
(45) Veröffentlichungstag und Bekanntmachung des Hinweises auf die Patenterteilung: **24.04.2024**
(21) Anmeldenummer: 21171216.1
(22) Anmeldetag: 29.04.2021
(51) Int. Cl.: A61B 6/06, A61B 6/03, A61B 6/00

(54) **CT-ANLAGE MIT EINEM KOLLIMATORBLECH UND VERFAHREN ZUM BEFESTIGEN EINES KOLLIMATORBLECHS EINER CT-ANLAGE**
CT SYSTEM WITH A COLLIMATOR PLATE AND METHOD FOR FIXING A COLLIMATOR PLATE OF A CT SYSTEM
INSTALLATION CT DOTÉE D'UNE TÔLE DE COLLIMATEUR ET PROCÉDÉ DE FIXATION D'UNE TÔLE DE COLLIMATEUR D'UNE INSTALLATION CT

(43) Veröffentlichungstag der Anmeldung: 25.05.2022
(73) Patentinhaber: Siemens Healthineers AG, 91301 Forchheim (DE)
(72) Erfinder: Berner, Matthias, 91074 Herzogenaurach (DE); Fehre, Jens, 91353 Hausen (DE)
(74) Vertreter: Siemens Healthineers Patent Attorneys

(56) Entgegenhaltungen:
- CN-U- 205 433 722
- JP-A- 2011 152 411
- US-A- 4 361 902
- US-A1- 2017 332 988

## Beschreibung

Die Erfindung betrifft eine CT-Anlage, also eine Computertomographie-Anlage, und ein Verfahren zum Befestigen eines Kollimatorblechs einer CT-Anlage.

Die vorliegende Erfindung geht aus von einer CT-Anlage,
- wobei die CT-Anlage eine Basisstruktur, eine Zusatzstruktur, eine Röntgenquelle, einen Röntgendetektor und ein Kollimatorblech umfasst,
- wobei die Zusatzstruktur an der Basisstruktur drehbar gelagert ist, so dass die Zusatzstruktur relativ zu der Basisstruktur um eine Rotationsachse rotierbar ist,
- wobei die Röntgenquelle und der Röntgendetektor an der Zusatzstruktur derart befestigt sind, dass sie relativ zueinander ortsfest angeordnet sind und sich zwischen der Röntgenquelle und dem Röntgendetektor im Bereich der Rotationsachse ein Untersuchungsbereich befindet, in dem ein Patient oder ein Körperteil eines Patienten angeordnet werden kann.

Derartige CT-Anlagen sind allgemein bekannt. Die Anordnung von Röntgenquelle, Röntgendetektor und Kollimatorblech ist generisch. Insbesondere können auch mehrere, beispielsweise zwei, derartige Gruppen vorhanden sein, die jeweils eine eigene Röntgenquelle, einen eigenen Röntgendetektor und ein eigenes Kollimatorblech umfassen. Die Rotationsachse und der Untersuchungsbereich sind jedoch für alle derartigen Gruppen dieselben.

Eine CT-Anlage besteht aus vielen verschiedenen Komponenten. Die Komponenten bestehen oftmals ihrerseits ebenfalls aus mehreren Einzelteilen. Derartige Komponenten werden zunächst für sich zusammengebaut und sodann getestet. Erst nach dem Testen werden diese Komponenten in die CT-Anlage als Ganzes eingebaut. Nach dem Einbauen in die CT-Anlage erfolgt ein erneuter Test der CT-Anlage als Ganzes.

Bei der Vorgehensweise des Standes der Technik ergeben sich ein hoher Montageaufwand und hiermit verbunden hohe Kosten.

US4361902 A offenbart eine CT Anlage gemäß Oberbegriff von Anspruch 1.

Fällt bei einer Komponente, die ihrerseits aus mehreren Einzelteilen besteht, eines der Einzelteile der Komponente aus, so kann es erforderlich sein, dass die gesamte Komponente ausgebaut wird, wobei es erst nach dem Ausbau der Komponente mit dem defekten Einzelteil möglich ist, das defekte Einzelteil auszutauschen.

Dies gilt insbesondere auch für eine im Stand der Technik vorhandene Kollimatoreinheit. Die Kollimatoreinheit umfasst einen Grundkörper, an dem das Kollimatorblech befestigt ist. An dem Grundkörper kann ferner zur Dosiserfassung ein Röntgensensor befestigt sein. Mit ihrem Grundkörper ist die Kollimatoreinheit lösbar an der Zusatzstruktur befestigt.

Die Vorgehensweise des Standes der Technik ist im Ergebnis sehr kostenintensiv und verbraucht unnötig viele Ressourcen. Die Aufgabe der vorliegenden Erfindung besteht darin, Möglichkeiten zu schaffen, mittels derer ein effizienterer Aufbau und hiermit verbunden eine effizientere Wartung und Reparatur einer CT-Anlage möglich sind.

Die Aufgabe wird jeweils gelöst durch eine CT-Anlage mit den Merkmalen des Anspruchs 1 und durch ein Verfahren mit den Merkmalen des Anspruchs 11. Vorteilhafte Ausgestaltungen der erfindungsgemäßen CT-Anlage sind Gegenstand der abhängigen Ansprüche.

Die Haltestruktur kann insbesondere zur lösbaren Aufnahme des Röntgensensors, beispielsweise zur formschlüssigen lösbaren Aufnahme des Röntgensensors, eingerichtet sein. Der Röntgensensor kann insbesondere zwischen der Röntgenquelle und dem Untersuchungsbereich derart lösbar in die Haltestruktur aufgenommen, beispielsweise formschlüssig aufgenommen, und an der Haltestruktur befestigt sein, dass der Röntgensensor als solcher von der Haltestruktur lösbar ist, insbesondere als solcher unabhängig von dem Kollimatorblech von der Haltestruktur lösbar ist.

Eine Ausführungsform sieht vor, dass das Kollimatorblech und ein Durchmesser des Untersuchungsbereichs auf dasselbe Körperteil des Patienten abgestimmt sind. Insbesondere kann hierfür eine Lage des Kollimatorblechs und/oder eine Durchtrittsöffnung des Kollimatorblechs für von der Röntgenquelle emittierte Röntgenstrahlung auf das Körperteil des Patientenabgestimmt sein. Der Durchmesser des Untersuchungsbereichs kann insbesondere eine zu der Rotationsachse senkrechte Erstreckung des Untersuchungsbereichs betreffen.

Insbesondere können eine zu der Rotationsachse parallele Abmessung der Durchtrittsöffnung des Kollimatorblechs und der Durchmesser des Untersuchungsbereichs auf dasselbe Körperteil des Patienten abgestimmt sein. Durch die zu der Rotationsachse parallele Abmessung der Durchtrittsöffnung des Kollimatorblechs kann insbesondere eine Schichtkollimation eines Fächerstrahls der von der Röntgenquelle emittierten Röntgenstrahlung bestimmt sein. Weiterhin kann vorgesehen sein, dass eine zu der Rotationsachse senkrechte Abmessung der Durchtrittsöffnung des Kollimatorblechs und der Durchmesser des Untersuchungsbereichs auf dasselbe Körperteil des Patienten abgestimmt sind.

Insbesondere kann vorgesehen sein, dass sich das Kollimatorblech flächig in einer Kollimatorblechfläche erstreckt. Die Kollimatorblechfläche kann beispielsweise flach oder gekrümmt sein. Die Kollimatorblechfläche kann insbesondere in einer Kollimatorblechebene liegen und/oder parallel zu der Rotationsachse sein.

Weiterhin kann vorgesehen sein, dass die zu der Rotationsachse parallele Abmessung der Durchtrittsöffnung des Kollimatorblechs eine zu der Rotationsachse parallele und zu der Kollimatorblechfläche parallele Erstreckung der Durchtrittsöffnung des Kollimatorblechs betrifft und/oder dass die zu der Rotationsachse senkrechte Abmessung der Durchtrittsöffnung des Kollimatorblechs eine zu der Rotationsachse senkrechte und zu der Kollimatorblechfläche parallele Erstreckung der Durchtrittsöffnung betrifft.

Durch die Abstimmung des Kollimatorblechs und des Durchmessers des Untersuchungsbereichs aufeinander ist - bezogen auf das Körperteil des Patienten - ein optimierter Betrieb der CT-Anlage möglich. Insbesondere kann der Bedarf an unterschiedlichen Durchtrittsöffnungen, die sich in Bezug auf die jeweilige zu der Rotationsachse parallele Abmessung der Durchtrittsöffnung unterscheiden, verringert sein, wenn aufgrund des Durchmessers des Untersuchungsbereichs die Verwendung der CT-Anlage für bestimmte Körperbereiche des Patienten ausgeschlossen werden kann.

Das Körperteil des Patienten kann insbesondere dessen Kopf sein. Insbesondere zu diesem Zweck - aber nicht notwendigerweise ausschließlich zu diesem Zweck - kann der Durchmesser des Untersuchungsbereichs kleiner als 45 cm, beispielsweise kleiner als 40 cm, und/oder größer als 25 cm, beispielsweise größer als 30 cm, sein. Der Untersuchungsbereich kann insbesondere in Form einer tunnelförmigen Öffnung ausgebildet sein.

Weiterhin kann vorgesehen sein, dass das Kollimatorblech im an der Zusatzstruktur befestigten Zustand relativ zur Röntgenquelle ortsfest angeordnet ist. Alternativ ist es möglich,
- dass das Kollimatorblech mehrere Durchtrittsöffnungen für von der Röntgenquelle emittierte Röntgenstrahlung aufweist,
- dass das Kollimatorblech an der Zusatzstruktur beweglich gelagert ist und
- dass die CT-Anlage einen an der Zusatzstruktur lösbar befestigten Kollimatorantrieb aufweist, mittels dessen das Kollimatorblech bewegbar ist.

In diesem Fall kann durch entsprechende Positionierung des Kollimatorblechs je nach Bedarf die eine oder die andere Durchtrittsöffnung im Strahlengang positioniert werden.

Eine Ausführungsform sieht vor, dass die Zusatzstruktur - gegebenenfalls mit Ausnahme der Bewegbarkeit des Kollimatorblechs als Ganzes - keine bewegbaren Elemente aufweist. Vielmehr ist die Zusatzstruktur nur als Ganzes relativ zu der Basisstruktur um die Rotationsachse rotierbar. Dadurch wird die Wahrscheinlichkeit, dass an der Zusatzstruktur Defekte irgendwelcher Art auftreten, deutlich reduziert. Insbesondere kann die Wahrscheinlichkeit, dass ein Defekt der Zusatzstruktur auftritt, deutlich kleiner sein als die Wahrscheinlichkeit, dass ein Defekt des Röntgensensors auftritt, und/oder deutlich kleiner sein als die Wahrscheinlichkeit, dass ein Defekt des Kollimatorantriebs auftritt.

Alternativ ist es möglich,
- dass die CT-Anlage (mindestens) einen Elementantrieb und ein mittels des Elementantriebs bewegbares Element aufweist,
- dass das bewegbare Element zwischen der Röntgenquelle und dem Untersuchungsbereich an der Zusatzstruktur befestigt ist und
- dass der Elementantrieb und das bewegbare Element von der Zusatzstruktur lösbar sind.

Dadurch ergibt sich eine erhöhte Flexibilität beim Betrieb der CT-Anlage, wobei dennoch auch der Elementantrieb und/oder das bewegbare Element bei Bedarf ausgetauscht werden können. Das bewegbare Element kann insbesondere ein Filter sein, mittels dessen die von der Röntgenquelle emittierte Röntgenstrahlung gefiltert wird.

Vorzugsweise ist auch im Falle eines Elementantriebs und eines bewegbaren Elements das Kollimatorblech von der Zusatzstruktur lösbar, während der Elementantrieb an der Zusatzstruktur befestigt ist und das bewegbare Element an der Zusatzstruktur gelagert ist. Es ist also nicht erforderlich, zunächst den Elementantrieb von der Zusatzstruktur zu lösen und/oder das bewegbare Element von der Zusatzstruktur zu entfernen und erst dann das Kollimatorblech zu entfernen. Vielmehr kann direkt das Kollimatorblech entfernt und gegebenenfalls durch ein anderes Kollimatorblech ersetzt werden.

Vorzugsweise ist auch im Falle eines Elementantriebs und eines bewegbaren Elements der Röntgensensor von der Zusatzstruktur lösbar, während der Elementantrieb an der Zusatzstruktur befestigt ist und das bewegbare Element an der Zusatzstruktur gelagert ist. Es ist also nicht erforderlich, zunächst den Elementantrieb von der Zusatzstruktur zu lösen und/oder das bewegbare Element von der Zusatzstruktur zu entfernen und erst dann den Röntgensensor zu entfernen. Vielmehr kann direkt der Röntgensensor entfernt und gegebenenfalls durch einen anderen Röntgensensor ersetzt werden.

In einer besonders bevorzugten Ausgestaltung ist die CT-Anlage als mobile CT-Anlage ausgebildet. In diesem Fall weist die Basisstruktur in einem Bodenbereich Räder auf, mittels derer die CT-Anlage auf einem Untergrund aufsteht und relativ zu dem Untergrund bewegbar gelagert ist.

Die Erfindung betrifft ferner ein Verfahren zum Befestigen eines Kollimatorblechs einer CT-Anlage,
- wobei die CT-Anlage eine Basisstruktur, eine Zusatzstruktur, eine Röntgenquelle, einen Röntgendetektor und das Kollimatorblech umfasst,
- wobei die Zusatzstruktur an der Basisstruktur drehbar gelagert ist, so dass die Zusatzstruktur relativ zu der Basisstruktur um eine Rotationsachse rotierbar ist,
- wobei die Röntgenquelle und der Röntgendetektor an der Zusatzstruktur derart befestigt sind, dass sie relativ zueinander ortsfest angeordnet sind und sich zwischen der Röntgenquelle und dem Röntgendetektor im Bereich der Rotationsachse ein Untersuchungsbereich befindet, in dem ein Patient oder ein Körperteil eines Patienten angeordnet werden kann,
**dadurch gekennzeichnet**,
dass das Kollimatorblech zwischen der Röntgenquelle und dem Untersuchungsbereich derart lösbar an der Zusatzstruktur befestigt wird, dass das Kollimatorblech als solches von der Zusatzstruktur gelöst werden kann, insbesondere derart von der Zusatzstruktur gelöst werden kann, dass die Zusatzstruktur dabei weder zerlegt noch zerstört wird.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der ein Verfahren betrifft, auch mit Merkmalen, die im Zusammenhang mit einer Vorrichtung beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, der durch die Ansprüche vorgegeben ist.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.
- FIG 1: schematisch eine CT-Anlage von vorne,
- FIG 2: die CT-Anlage von FIG 1 von der Seite,
- FIG 3: eine Haltestruktur von unten,
- FIG 4: eine weitere Haltestruktur von unten und
- FIG 5: eine weitere Haltestruktur von der Seite.

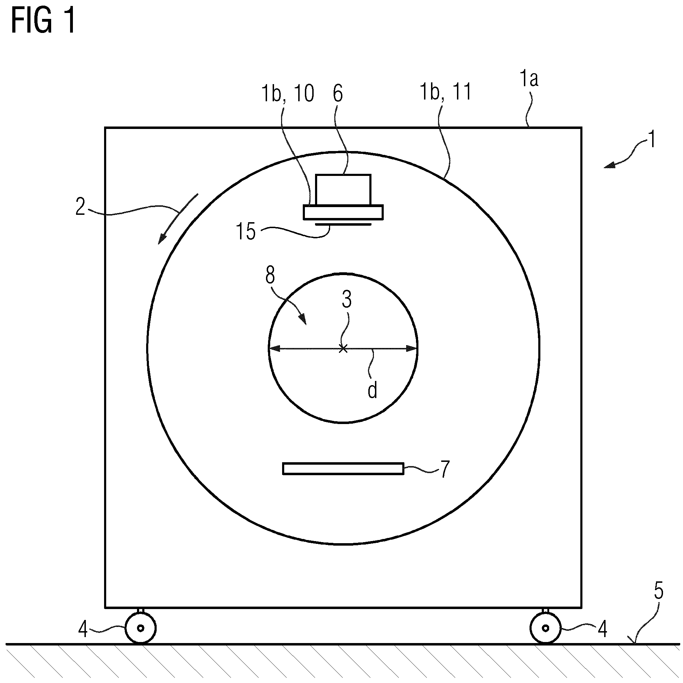

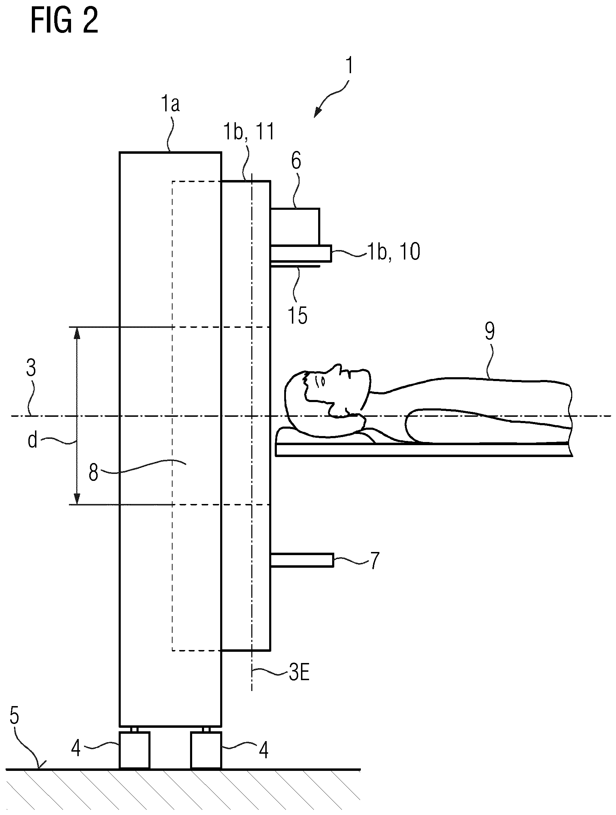

Gemäß den FIG 1 und 2 umfasst eine CT-Anlage 1 eine Basisstruktur 1a und eine Zusatzstruktur 1b. Wie in FIG 1 durch einen Pfeil 2 angedeutet ist, ist die Zusatzstruktur 1b an der Basisstruktur 1a drehbar gelagert, so dass die Zusatzstruktur 1b relativ zu der Basisstruktur 1a um eine Rotationsachse 3 rotierbar ist. Die Zusatzstruktur 1b wird oftmals als Disk oder Drum bezeichnet. Die Zusatzstruktur 1b kann insbesondere einstückig ausgebildet sein, beispielsweise als einziges Teil, das nicht weiter zerlegt werden kann, ohne es zu zerstören. Beispielsweise kann die Zusatzstruktur 1b als Gussteil ausgebildet sein.

Die CT-Anlage 1 kann ortsfest angeordnet sein. Alternativ kann die CT-Anlage 1 mobil sein. Im letztgenannten Fall weist die Basisstruktur 1a in einem Bodenbereich Räder 4 auf, mittels derer die CT-Anlage 1 auf einem Untergrund 5 aufsteht und relativ zu dem Untergrund 5 bewegbar gelagert ist.

Die CT-Anlage 1 umfasst ferner eine Röntgenquelle 6 und einen Röntgendetektor 7. Die Röntgenquelle 6 kann als übliche Röntgenquelle ausgebildet sein. Der Röntgendetektor 7 dient der Bildgebung. Er ist oftmals als Zeilendetektor oder als Flächendetektor ausgebildet. Die Röntgenquelle 6 und der Röntgendetektor 7 sind an der Zusatzstruktur 1b befestigt. Im befestigten Zustand sind die Röntgenquelle 6 und der Röntgendetektor 7 relativ zueinander ortsfest angeordnet. Die Röntgenquelle 6 und der Röntgendetektor 7 sind somit in diesem Fall zusammen mit der Zusatzstruktur 1b um die Rotationsachse 3 drehbar. Relativ zueinander bleiben sie aber dennoch ortsfest.

Zwischen der Röntgenquelle 6 und dem Röntgendetektor 7 befindet sich im Bereich der Rotationsachse 3 ein Untersuchungsbereich 8 in Form einer tunnelförmigen Öffnung. Im Untersuchungsbereich 8 kann - siehe FIG 2 - ein Patient 9 oder ein Körperteil eines Patienten 9 (beispielsweise dessen Kopf) angeordnet werden.

Die Zusatzstruktur 1b weist eine Haltestruktur 10 auf, wobei die Haltestruktur 10 zur lösbaren Aufnahme des Kollimatorblechs 15 eingerichtet ist. Das Kollimatorblech 15 ist zwischen der Röntgenquelle 6 und dem Untersuchungsbereich 8 derart lösbar in die Haltestruktur 10 aufgenommen und an der Haltestruktur 10 befestigt, dass das Kollimatorblech 15 als solches von der Haltestruktur 1b lösbar ist.

Die Zusatzstruktur 1b weist ferner einen ringförmigen Drehrahmen 11 auf, der sich flächig in einer Rotationsebene 3E erstreckt. Die Rotationsebene 3E ist senkrecht zu der Rotationsachse 3. Die Haltestruktur 10 ragt im Wesentlichen senkrecht zu der Rotationsebene 3E relativ zu dem ringförmigen Drehrahmen 11 heraus.

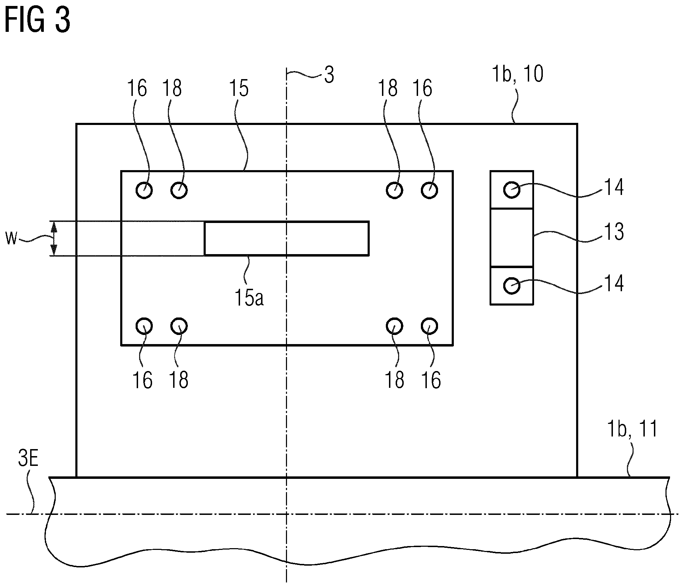

Die CT-Anlage umfasst ferner ein Kollimatorblech 15. Das Kollimatorblech 15 ist zwischen der Röntgenquelle 6 und dem Untersuchungsbereich 8 an der Zusatzstruktur 1b befestigt. Beispielsweise kann die Zusatzstruktur 1b eine Haltestruktur 10 aufweisen, an welcher die Röntgenquelle 6 befestigt ist. Die Haltestruktur 10 ist in diesem Fall Bestandteil der Zusatzstruktur 1b, beispielsweise ein unlösbarer Bestandteil des Gussteils. Im Falle der Haltestruktur 10 kann das Kollimatorblech 15 beispielsweise - siehe ergänzend FIG 3 - auf der Unterseite der Haltestruktur 10 befestigt sein, also auf der von der Röntgenquelle 6 abgewandten Seite der Haltestruktur 10, so dass die Haltestruktur 10 zwischen der Röntgenquelle 6 und dem Kollimatorblech 15 angeordnet ist.

Das Kollimatorblech 15 ist von der Zusatzstruktur 1b lösbar, und zwar als solches. Zum Lösen des Kollimatorblechs 15 von der Zusatzstruktur 1b wird also nicht ein Grundkörper, der zur Aufnahme des Kollimatorblechs 15 eingerichtet ist, von der Zusatzstruktur 1b gelöst, sondern das Kollimatorblech 15 selbst. Es kann zwar erforderlich sein, vor dem Lösen des Kollimatorblechs 15 eine Verkleidung der CT-Anlage zumindest teilweise zu entfernen. Dennoch erfolgt das Lösen des Kollimatorblechs 15 als solches, ohne dass ein Lösen eines Grundkörpers, der zur Aufnahme des Kollimatorblechs 15 eingerichtet ist, von der Zusatzstruktur 1b erfolgt.

Das Kollimatorblech 15 kann beispielsweise mittels Befestigungsschrauben 16 an der Zusatzstruktur 1b befestigt sein. Für eine exakte Positionierung des Kollimatorblechs 15 relativ zur Zusatzstruktur 1b (und damit auch relativ zur Röntgenquelle 6) kann das Kollimatorblech 15 beispielsweise entsprechend der Darstellung in FIG 3 Ausnehmungen aufweisen, die mit Zentrierzapfen 18 (oder ähnlichen Positionierhilfen) der Zusatzstruktur 1b zusammenwirken.

Die Zusatzstruktur 1b weist in aller Regel ferner einen Röntgensensor 13 auf. Der Röntgensensor 13 dient - im Gegensatz zum Röntgendetektor 7 - nicht der Bildgebung. Er dient vielmehr der Dosismessung. Der Röntgensensor 13 ist - beispielsweise mittels Befestigungsschrauben 14 - lösbar an der Zusatzstruktur 1b befestigt. Der Röntgensensor 13 ist ebenfalls derart befestigt, dass er sich zwischen der Röntgenquelle 6 und dem Untersuchungsbereich 8 befindet. Weiterhin ist der Röntgensensor 13 - analog zum Kollimatorblech 15 - als solcher von der Zusatzstruktur 1b lösbar. Der Röntgensensor 13 kann beispielsweise mittels Befestigungsschrauben 14 ebenfalls auf der Unterseite der Haltestruktur 10 befestigt sein. In diesem Fall müssen zum Lösen des Röntgensensors 13 von der Zusatzstruktur 1b lediglich die Befestigungsschrauben 14 gelöst werden.

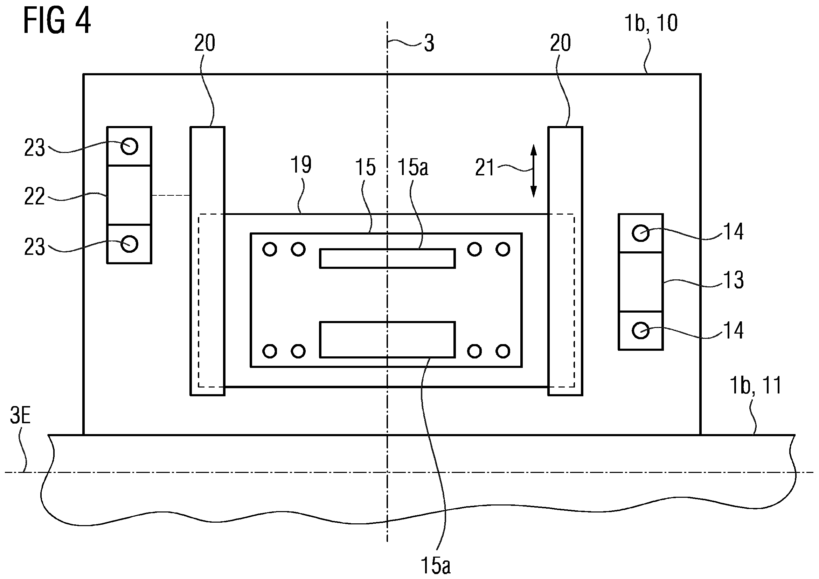

Es ist, wie in FIG 3 dargestellt, möglich, dass das Kollimatorblech 15, wenn es an der Zusatzstruktur 1b befestigt ist relativ zur Röntgenquelle 6 ortsfest angeordnet ist. In diesem Fall weist das Kollimatorblech 15 nur eine einzelne Durchtrittsöffnung 15a für von der Röntgenquelle 6 emittierte Röntgenstrahlung auf. Die Durchtrittsöffnung 15a weist in der Regel die Form eines Rechtecks auf, meist sogar die Form eines recht schmalen Rechtecks, also eines Schlitzes. Es ist jedoch entsprechend der Darstellung in FIG 4 alternativ möglich, dass das Kollimatorblech 15 an der Zusatzstruktur 1b beweglich gelagert ist. Beispielsweise kann das Kollimatorblech 15 auf einem Schlitten 19 (oder einem ähnlichen Element) befestigt sein, wobei der Schlitten 19 in einer Führung 20 geführt wird, so dass er linear verschoben werden kann. Die Richtung, in welcher der Schlitten 19 verschiebbar ist, ist in FIG 4 durch einen Pfeil 21 angedeutet.

Das Bewegen des Kollimatorblechs 15 erfolgt durch einen Kollimatorantrieb 22. Dies ist in FIG 4 durch eine gestrichelte Linie vom Kollimatorantrieb 22 zur Führung 20 angedeutet. Der Kollimatorantrieb 22 ist lösbar an der Zusatzstruktur 1b befestigt, beispielsweise mittels Befestigungsschrauben 23. Mittels des Kollimatorantriebs 22 kann das Kollimatorblech 15 in mehrere definierte Positionen verfahren werden. Ferner weist das Kollimatorblech 15 in diesem Fall mehrere Durchtrittsöffnungen 15a auf. Je nach Positionierung des Kollimatorblechs 15 tritt die Röntgenstrahlung der Röntgenquelle 6 durch die eine oder die andere der Durchtrittsöffnungen 15a hindurch.

Das Kollimatorblech 15, insbesondere die zu der Rotationsachse 3 parallele Abmessung w der Durchtrittsöffnung 15a des Kollimatorblechs 15, und der Durchmesser d des Untersuchungsbereichs 8 senkrecht zu der Rotationsachse 3 sind auf dasselbe Körperteil des Patienten 9 abgestimmt, wobei es sich bei dem Körperteil des Patienten 9 um den Kopf des Patienten 9 handelt.

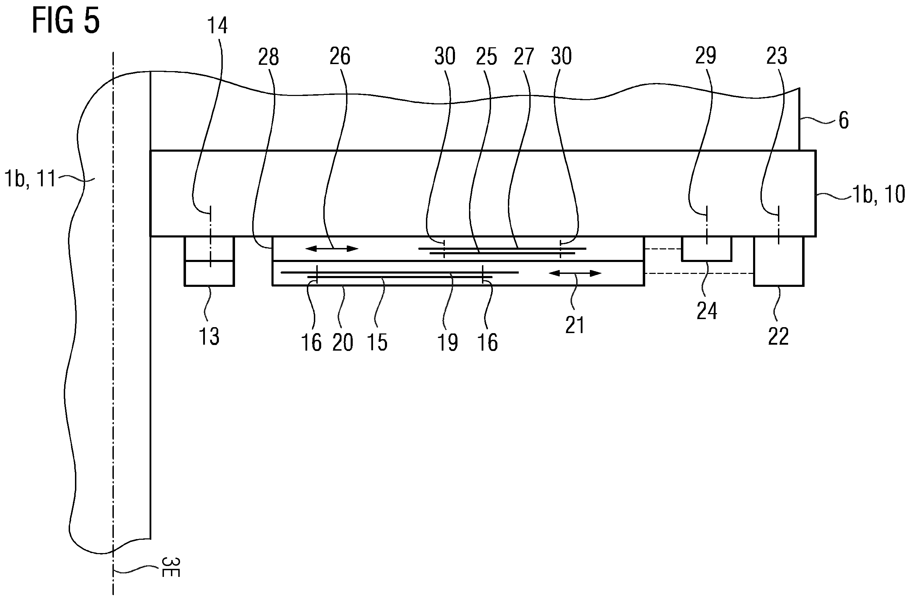

In der Ausgestaltung gemäß FIG 5 ist an der Zusatzstruktur 1b, insbesondere an der Haltestruktur 10, die ein Bestandteil der Zusatzstruktur 1b ist, weiterhin auch ein mittels eines Elementantriebs 24 bewegbares Element 25 befestigt. Das bewegbare Element 25 kann beispielsweise analog zu dem Kollimatorblech 15 in einem Schlitten 27 gelagert sein, der seinerseits in einer Führung 28 geführt wird. Das bewegbare Element 25 ist zwischen der Röntgenquelle 6 und dem Untersuchungsbereich 8 befestigt. Es kann beispielsweise als Filter zum Filtern der Röntgenstrahlung ausgebildet sein.

Das bewegbare Element 25 kann mittels des Elementantriebs 24 beispielsweise in Richtung eines Pfeils 26 bewegt werden. Der Pfeil 26 ist in aller Regel parallel zum Pfeil 21. Der Elementantrieb 24 ist ebenfalls an der Zusatzstruktur 1b befestigt. Der Elementantrieb 24 ist vorzugsweise an der Zusatzstruktur 1b lösbar befestigt. Beispielsweise kann eine Befestigung mittels Befestigungsschrauben 29 erfolgen. Alternativ oder zusätzlich zu einer lösbaren Befestigung des Elementantriebs 24 kann das bewegbare Element 25 von der Zusatzstruktur 1b lösbar sein, beispielsweise durch ein Lösen von Befestigungsschrauben 30.

Der Elementantrieb 24 und das bewegbare Element 25 sind vorzugsweise derart angeordnet, dass sie das Lösen des Röntgensensors 13 und/oder des Kollimatorblechs 15 von der Zusatzstruktur 1b nicht behindern. Der Röntgensensor 13 und/oder das Kollimatorblech 15 sind vorzugsweise also auch von der Zusatzstruktur 1b lösbar, während der Elementantrieb 24 und/oder das bewegbare Element 25 an der Zusatzstruktur 1b befestigt sind. Beispielsweise kann dies dadurch erreicht werden, dass von der Röntgenquelle 6 zum Röntgendetektor 7 gesehen zunächst die Haltestruktur 10, sodann das bewegbare Element 25 und erst danach das Kollimatorblech 15 aufeinander folgen. Weiterhin ist es auch im Falle der umgekehrten Anordnung, bei welcher von der Röntgenquelle 6 zum Röntgendetektor 7 gesehen zunächst die Haltestruktur 10, sodann das Kollimatorblech 15 und erst danach das bewegbare Element 25 aufeinander folgen, möglich, dass das Kollimatorblech 15 und/oder das bewegbare Element 25 derart verfahren werden, dass das Kollimatorblech 15 zugänglich ist, obwohl das bewegbare Element 25 nicht gelöst wurde.

Die CT-Anlage umfasst eine Basisstruktur 1a, eine Zusatzstruktur 1b, eine Röntgenquelle 6, einen Röntgendetektor 7 und ein Kollimatorblech 15. Die Zusatzstruktur 1b ist an der Basisstruktur 1a drehbar gelagert, so dass die Zusatzstruktur 1b relativ zu der Basisstruktur 1a um eine Rotationsachse 3 rotierbar ist. Die Röntgenquelle 6 und der Röntgendetektor 7 sind an der Zusatzstruktur 1b derart befestigt, dass sie relativ zueinander ortsfest angeordnet sind und sich zwischen der Röntgenquelle 6 und dem Röntgendetektor 7 im Bereich der Rotationsachse 3 ein Untersuchungsbereich 8 befindet, in dem ein Patient 9 oder ein Körperteil eines Patienten 9 angeordnet werden kann. Das Kollimatorblech 15 ist zwischen der Röntgenquelle 6 und dem Untersuchungsbereich 8 lösbar an der Zusatzstruktur 1b befestigt. Es ist als solches von der Zusatzstruktur 1b lösbar.

Die vorliegende Erfindung weist insbesondere den Vorteil auf, dass ein direkter, unmittelbarer Ausbau insbesondere des Kollimatorblechs 15 und gegebenenfalls auch weiterer Komponenten wie beispielsweise des Röntgensensors 13 möglich ist. Besonders vorteilhaft ist dies, wenn die verschiedenen Komponenten unabhängig voneinander ausgetauscht werden können, wenn also - mit Ausnahme der Verkleidung - eine bestimmte Komponente ausgetauscht werden kann, ohne zuvor andere Komponenten lösen und hinterher wieder befestigen zu müssen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. CT-Anlage (1),
- wobei die CT-Anlage (1) eine Basisstruktur (1a), eine Zusatzstruktur (1b), eine Röntgenquelle (6), einen Röntgendetektor (7) und ein Kollimatorblech (15) umfasst,
- wobei die Zusatzstruktur (1b) an der Basisstruktur (1a) drehbar gelagert ist, so dass die Zusatzstruktur (1b) relativ zu der Basisstruktur (1a) um eine Rotationsachse (3) rotierbar ist,
- wobei die Zusatzstruktur (1b) einen ringförmigen Drehrahmen (11) aufweist, der sich flächig in einer Rotationsebene (3E) erstreckt, wobei die Rotationsebene (3E) senkrecht zu der Rotationsachse (3) ist,
- wobei die Zusatzstruktur (1b) eine Haltestruktur (10) aufweist, wobei die Haltestruktur (10) zur lösbaren Aufnahme des Kollimatorblechs (15) eingerichtet ist,
- wobei die Röntgenquelle (6) und der Röntgendetektor (7) an der Zusatzstruktur (1b) derart befestigt sind, dass sie relativ zueinander ortsfest angeordnet sind und sich zwischen der Röntgenquelle (6) und dem Röntgendetektor (7) im Bereich der Rotationsachse (3) ein Untersuchungsbereich (8) befindet, in dem ein Patient (9) oder ein Körperteil eines Patienten (9) angeordnet werden kann,
- wobei das Kollimatorblech (15) zwischen der Röntgenquelle (6) und dem Untersuchungsbereich (8) derart lösbar an der Zusatzstruktur (1b) befestigt ist, dass das Kollimatorblech (15) als solches von der Zusatzstruktur (1b) lösbar ist,
- wobei das Kollimatorblech (15) zwischen der Röntgenquelle (6) und dem Untersuchungsbereich (8) derart lösbar in die Haltestruktur (10) aufgenommen und an der Haltestruktur (10) befestigt ist, dass das Kollimatorblech (15) als solches von der Haltestruktur (1b) lösbar ist,
**dadurch gekennzeichnet,**
**dass** die Zusatzstruktur (1b) einstückig und als Gussteil ausgebildet ist und dass die Haltestruktur (10) relativ zu dem ringförmigen Drehrahmen (11) im Wesentlichen senkrecht zu der Rotationsebene (3E) herausragt, wobei die Haltestruktur Bestandteil der Zusatzstruktur ist.

2. CT-Anlage (1) nach einem der obigen Ansprüche,
**dadurch gekennzeichnet,**
**dass** die CT-Anlage (1) ferner einen Röntgensensor (13) aufweist und
**dass** der Röntgensensor (13) zwischen der Röntgenquelle (6) und dem Untersuchungsbereich (8) derart lösbar an der Zusatzstruktur (1b) befestigt ist, dass der Röntgensensor (13) als solcher von der Zusatzstruktur (1b) lösbar ist.

3. CT-Anlage (1) nach einem der Ansprüche 1 bis 2,
**dadurch gekennzeichnet,**
**dass** das Kollimatorblech (15) und ein Durchmesser (d) des Untersuchungsbereichs (8) auf dasselbe Körperteil des Patienten (9) abgestimmt sind.

4. CT-Anlage (1) nach Anspruch 3,
**dadurch gekennzeichnet,**
**dass** das Körperteil des Patienten (9) dessen Kopf ist.

5. CT-Anlage (1) nach Anspruch 3 oder 4,
**dadurch gekennzeichnet,**
**dass** der Durchmesser (d) des Untersuchungsbereichs (8) kleiner als 45 cm ist.

6. CT-Anlage (1) nach einem der Ansprüche 1 bis 5,
**dadurch gekennzeichnet,**
**dass** das Kollimatorblech (15) in einem an der Zusatzstruktur (1b) befestigten Zustand relativ zur Röntgenquelle (6) ortsfest angeordnet ist.

7. CT-Anlage (1) nach einem der Ansprüche 1 bis 6,
**dadurch gekennzeichnet,**
- **dass** das Kollimatorblech (15) mehrere Durchtrittsöffnungen (15a) für von der Röntgenquelle (6) emittierte Röntgenstrahlung aufweist,
- **dass** das Kollimatorblech (15) an der Zusatzstruktur (1b) beweglich gelagert ist und
- **dass** die CT-Anlage (1) einen an der Zusatzstruktur (1b) lösbar befestigten Kollimatorantrieb (22) aufweist, mittels dessen das Kollimatorblech (15) bewegbar ist.

8. CT-Anlage (1) nach einem der obigen Ansprüche,
**dadurch gekennzeichnet,**
- **dass** die CT-Anlage (1) einen Elementantrieb (24) und ein mittels des Elementantriebs (24) bewegbares Element (25) aufweist,
- **dass** das bewegbare Element (25) zwischen der Röntgenquelle (6) und dem Untersuchungsbereich (8) an der Zusatzstruktur (1b) befestigt ist und
- **dass** der Elementantrieb (24) und das bewegbare Element (25) von der Zusatzstruktur (1b) lösbar sind.

9. CT-Anlage (1) nach Anspruch 8,
**dadurch gekennzeichnet,**
**dass** das Kollimatorblech (15) von der Zusatzstruktur (1b) lösbar ist, während der Elementantrieb (24) an der Zusatzstruktur (1b) befestigt ist und das bewegbare Element (25) an der Zusatzstruktur (1b) gelagert ist.

10. CT-Anlage (1) nach einem der obigen Ansprüche,
**dadurch gekennzeichnet,**
**dass** die Basisstruktur (1a) in einem Bodenbereich Räder (4) aufweist, mittels derer die CT-Anlage (1) auf einem Untergrund (5) aufsteht und relativ zu dem Untergrund (5) bewegbar gelagert ist.

11. Verfahren zum Befestigen eines Kollimatorblechs (15) einer CT-Anlage (1),
- wobei die CT-Anlage (1) nach einem der obigen Ansprüche ausgebildet ist,
**dadurch gekennzeichnet,**
**dass** das Kollimatorblech (15) zwischen der Röntgenquelle (6) und dem Untersuchungsbereich (8) derart lösbar an der Zusatzstruktur (1b) befestigt wird, dass das Kollimatorblech (15) als solches von der Zusatzstruktur (1b) gelöst werden kann.

## Claims

1. CT system (1),
- wherein the CT system (1) comprises a base structure (1a), an additional structure (1b), an X-ray source (6), an X-ray detector (7) and a collimator plate (15),
- wherein the additional structure (1b) is mounted on the base structure (1a) such that it can be rotated, so that the additional structure (1b) can be rotated relative to the base structure (1a) about an axis of rotation (3),
- wherein the additional structure (1b) has an annular rotating frame (11), which extends in a planar manner in a rotation plane (3E), wherein the rotation plane (3E) is perpendicular to the axis of rotation (3),
- wherein the additional structure (1b) has a holding structure (10), wherein the holding structure (10) is configured to accommodate the collimator plate (15) in a detachable manner,
- wherein the X-ray source (6) and the X-ray detector (7) are fastened to the additional structure (1b) in such a manner that they are arranged at a fixed position relative to one another and, between the X-ray source (6) and the X-ray detector (7) in the region of the axis of rotation (3), there is an examination region (8), in which a patient (9) or a body part of a patient (9) can be arranged,
- wherein the collimator plate (15) is fastened in a detachable manner to the additional structure, between the X-ray source (6) and the examination region (8), in such a manner that the collimator plate (15) can be detached from the additional structure (1b) as such,
- wherein the collimator plate (15), between the X-ray source (6) and the examination region (8), is accommodated in the holding structure (10) in a detachable manner and fastened to the holding structure (10) such that the collimator plate (15) can be detached from the holding structure (1b) as such,
**characterised in that**
the additional structure (1b) is embodied in one piece and as a cast part, and the holding structure (10) protrudes substantially perpendicularly in relation to the rotation plane (3E) relative to the annular rotating frame (11), wherein the holding structure is a constituent part of the additional structure.

2. CT system (1) according to one of the above claims,
**characterised in that**
the CT system (1) further has an X-ray sensor (13) and
the X-ray sensor (13) is fastened in a detachable manner to the additional structure (1b), between the X-ray source (6) and the examination region (8), in such a manner that the X-ray sensor (13) can be detached from the additional structure (1b) as such.

3. CT system (1) according to one of claims 1 to 2,
**characterised in that**
the collimator plate (15) and a diameter (d) of the examination region (8) are aligned with the same body part of the patient (9).

4. CT system (1) according to claim 3,
**characterised in that**
the body part of the patient (9) is the head thereof.

5. CT system (1) according to claim 3 or 4,
**characterised in that**
the diameter (d) of the examination region (8) is less than 45 cm.

6. CT system (1) according to one of claims 1 to 5,
**characterised in that**
the collimator plate (15) is arranged at a fixed position relative to the X-ray source (6) in a state in which it is fastened to the additional structure (1b).

7. CT system (1) according to one of claims 1 to 6,
**characterised in that**
- the collimator plate (15) has multiple passage openings (15a) for X-ray radiation emitted by the X-ray source (6),
- the collimator plate (15) is mounted on the additional structure (1b) such that it can move and
- the CT system (1) has a collimator drive (22) that is fastened to the additional structure (1b) in a detachable manner and by means of which the collimator plate (15) can be moved.

8. CT system (1) according to one of the above claims,
**characterised in that**
- the CT system (1) has an element drive (24) and an element (25) that can be moved by means of the element drive (24),
- the movable element (25) is fastened to the additional structure (1b) between the X-ray source (6) and the examination region (8) and
- the element drive (24) and the movable element (25) can be detached from the additional structure (1b).

9. CT system (1) according to claim 8,
**characterised in that**
the collimator plate (15) can be detached from the additional structure (1b) while the element drive (24) is fastened to the additional structure (1b) and the movable element (25) is mounted on the additional structure (1b).

10. CT system (1) according to one of the above claims,
**characterised in that**
the base structure (1a), in a bottom region, has wheels (4) by means of which the CT system (1) stands on a subsurface (5) and is mounted such that it can move relative to the subsurface (5).

11. Method for fastening a collimator plate (15) of a CT system (1),
- wherein the CT system (1) is embodied according to one of the above claims,
**characterised in that**
the collimator plate (15) is fastened in a detachable manner to the additional structure (1b), between the X-ray source (6) and the examination region (8), in such a manner that the collimator plate (15) can be detached from the additional structure (1b) as such.

## Revendications

1. Installation CT (1),
- dans laquelle l'installation CT (1) comprend une structure (1a) de base, une structure (1b) supplémentaire, une source (6) de rayons X, un détecteur (7) de rayons X et une tôle (15) de collimateur,
- dans laquelle la structure (1b) supplémentaire est montée tournante sur la structure (1a) de base, de manière à ce que la structure (1b) supplémentaire puisse tourner par rapport à la structure (1a) de base autour d'un axe (3) de rotation,
- dans laquelle la structure (1b) supplémentaire a un cadre (11) annulaire tournant, qui s'étend en surface dans un plan (3E) de rotation, le plan (3E) de rotation étant perpendiculaire à l'axe (3) de rotation,
- dans laquelle la structure (1b) supplémentaire a une structure (10) de maintien, la structure (10) de maintien étant agencée pour la réception amovible de la tôle (15) de collimateur,
- dans laquelle la source (6) de rayons X et le détecteur (7) de rayons X sont fixés à la structure (1b) supplémentaire, de manière à être montés fixes en position relativement l'un à l'autre et à ce que, entre la source (6) de rayons X et le détecteur (7) de rayons X, se trouve, dans la région de l'axe (3) de rotation, une région (8) d'examen, dans laquelle un patient (9) ou une partie du corps d'un patient (9) peut être disposé,
- dans laquelle la tôle (15) de collimateur est fixée de manière amovible à la structure (1b) supplémentaire entre la source (6) de rayons X et la partie (8) d'examen, de manière à ce que la tôle (15) de collimateur puisse, en tant que telle, être détachée de la structure (1b) supplémentaire,
- dans laquelle la tôle (15) de collimateur est reçue de manière amovible dans la structure (10) de maintien entre la source (6) de rayons X et la partie (8) d'examen et est fixée à la structure (10) de maintien, de manière à ce que la tôle (15) de collimateur, en tant que telle, puisse être détachée de la structure (1b) supplémentaire,
**caractérisée**
**en ce que** la structure (1b) supplémentaire est constituée d'une seule pièce et sous la forme d'une partie coulée et en ce que la structure (10) de maintien fait, sensiblement perpendiculairement au plan (3E) de rotation, saillie du cadre (11) annulaire tournant, la structure de maintien étant une partie constitutive de la structure supplémentaire.

2. Installation CT (1) suivant l'une des revendications précédentes,
**caractérisée**
**en ce que** l'installation CT (1) a en outre, un capteur (13) de rayons X et
**en ce que** le capteur (13) de rayons X est fixé de manière amovible à la structure (1b) supplémentaire entre la source (6) de rayons X et la partie (8) d'examen, de manière à ce que le capteur (13) de rayons X, en tant que tel, puisse être détaché de la structure (1b) supplémentaire.

3. Installation CT (1) suivant l'une des revendications 1 à 2,
**caractérisée**
**en ce que** la tôle (15) de collimateur et un diamètre (d) de la partie (8) d'examen sont adaptés à la même partie du corps du patient (9).

4. Installation CT (1) suivant la revendication 3,
**caractérisée**
**en ce que** la partie du corps du patient (9) en est la tête.

5. Installation CT (1) suivant la revendication 3 ou 4,
**caractérisée**
**en ce que** le diamètre (d) de la partie (8) d'examen est plus petit que 45 cm.

6. Installation CT (1) suivant l'une des revendications 1 à 5,
**caractérisée**
**en ce que** la tôle (15) de collimateur est, dans un état fixée à la structure (1b) supplémentaire, montée fixe en position par rapport à la source (6) de rayons X.

7. Installation CT (1) suivant l'une des revendications 1 à 6,
**caractérisée**
- **en ce que** la tôle (15) de collimateur a plusieurs ouvertures (15a) de passage du rayonnement X émis par la source (6) de rayons X,
- **en ce que** la tôle (15) de collimateur est montée mobile sur la structure (1b) supplémentaire et
- **en ce que** l'installation CT (1) a un entraînement (22) de collimateur, qui est fixé de manière amovible à la structure (1b) supplémentaire et au moyen duquel la tôle (15) de collimateur peut être déplacée.

8. Installation CT (1) suivant l'une des revendications précédentes,
**caractérisée**
- **en ce que** l'installation CT (1) a un entraînement (24) d'élément et un élément (25) pouvant être déplacé au moyen de l'entraînement (24) d'élément et
- **en ce que** l'élément (25) mobile est fixé à la structure (1b) supplémentaire entre la source (6) de rayons X et la partie (8) d'examen et
- **en ce que** l'entraînement (24) d'élément et l'élément (25) mobile peuvent être détachés de la structure (1b) supplémentaire.

9. Installation CT (1) suivant la revendication 8,
**caractérisée**
**en ce que** la tôle (15) de collimateur peut être détachée de la structure (1b) supplémentaire, tandis que l'entraînement (24) d'élément est fixé à la structure (1b) supplémentaire et que l'élément (25) mobile est monté sur la structure (1b) supplémentaire.

10. Installation CT (1) suivant l'une des revendications précédentes,
**caractérisée**
**en ce que** la structure (1a) de base a, dans une partie du fond, des roues (4), au moyen desquelles l'installation CT (1) s'élève d'un soubassement (5) et est montée mobile par rapport au soubassement (5).

11. Procédé de fixation d'une tôle (15) de collimateur par une installation CT (1),
- dans lequel l'installation CT (1) est constituée suivant l'une des revendications précédentes,
**caractérisé**
**en ce que** l'on fixe la tôle (15) de collimateur entre la source (6) de rayons X et la partie (8) d'examen, de manière détachable à la structure (1b) supplémentaire, de manière à pouvoir détacher la tôle (15) de collimateur, en tant que telle, de la structure (1b) supplémentaire.
